(19) Europäisches Patentamt
European Patent Office
Office européen des brevets

(11) Veröffentlichungsnummer: **0 006 587**
**B1**

(12) **EUROPÄISCHE PATENTSCHRIFT**

(45) Veröffentlichungstag der Patentschrift: **11.11.81**

(21) Anmeldenummer: **79102066.2**

(22) Anmeldetag: **22.06.79**

(51) Int. Cl.³: **C 07 D 209/46,**
A 61 K 31/40,
A 61 K 31/47,
A 61 K 31/64,
C 07 D 217/24

# (54) Sulfonylharnstoffe, Verfahren zu ihrer Herstellung sowie pharmazeutische Präparate auf Basis dieser Verbindungen.

(30) Priorität: **27.06.78 DE 2828079**

(43) Veröffentlichungstag der Anmeldung:
**09.01.80 Patentblatt 80/1**

(45) Bekanntmachung des Hinweises auf die Patenterteilung:
**11.11.81 Patentblatt 81/45**

(84) Benannte Vertragsstaaten:
**AT BE CH DE FR GB IT LU NL SE**

(56) Entgegenhaltungen:
DE - A - 1 445 774
DE - A - 1 670 660
DE - A - 1 670 700
DE - A - 2 000 339
DE - A - 2 157 607

(73) Patentinhaber: **HOECHST AKTIENGESELLSCHAFT**
**Postfach 80 03 20**
**D-6230 Frankfurt/Main 80 (DE)**

(72) Erfinder: **Weyer, Rudi, Dr.**
**Johann-Strauss-Strasse 43**
**D-6233 Kelkheim (Taunus) (DE)**
Erfinder: **Hitzel, Volker, Dr.**
**Kantstrasse 1b**
**D-6238 Hofheim an Taunus (DE)**
Erfinder: **Geisen, Karl, Dr.**
**Jahnstrasse 43**
**D-6000 Frankfurt/Main 71 (DE)**
Erfinder: **Regitz, Günter, Dr.**
**Dachbergstrasse 68**
**D-6232 Bad Soden am Tanus (DE)**

EP 0 006 587 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Sulfonylharnstoffe, Verfahren zu ihrer Herstellung sowie pharmazeutische Präparate auf Basis dieser Verbindungen

Die Erfindung betrifft Sulfonylharnstoffe der Formel

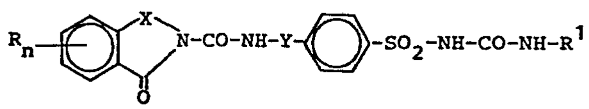

die als Substanz oder in Form ihrer physiologisch verträglichen Salze blutzuckersenkende Eigenschaften besitzen und sich durch starke und langanhaltende Senkung des Blutzuckerspiegels auszeichnen und daher als Arzneimittel verwendet werden können.

In der Formel bedeuten:

n 1 oder 2

R Wasserstoff, Alkyl mit 1 bis 4 Kohlenstoffatomen, Alkoxy mit 1 bis 4 Kohlenstoffatomen oder Halogen, wobei, falls n 2 ist, die R gleich oder verschieden sein können

X eine $—CH_2—$, $—CH_2—CH_2—$ oder

$$\begin{array}{c} CH_3 \\ | \\ —CH— \end{array}$$ Gruppe

Y Alkylen mit 2 oder 3 C-Atomen

$R^1$ Alkyl von 2 bis 8 C-Atomen, Cycloalkyl, Alkylcycloalkyl, Dialkylcycloalkyl, Cycloalkylalkyl, Cycloalkenyl, Alkylcycloalkenyl mit jeweils 4 bis 9 C-Atomen, Methylcyclopentylmethyl Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl, Benzyl.

In der allgemeinen Formel bedeutet R vorzugsweise Wasserstoff, Methyl und Halogen, insbesondere Chlor. Im Falle der Disubstitution sind die Dichlorverbindungen bevorzugt. Besonders bevorzugt ist für R Wasserstoff. X bedeutet vorzugsweise die $—CH_2$-Gruppe und Y vorzugsweise $—CH_2—CH_2—$ oder

$$\begin{array}{l} —CH—CH_2, \\ \;\;\;| \\ \;\;CH_3 \end{array}$$

wobei die $—CH_2—CH_2$-Gruppe besonders bevorzugt ist. $R^1$ ist vorzugsweise Butyl, Isobutyl, Methylcyclopentyl, Cyclopentylmethyl, Cyclohexyl, besonders bevorzugt sind Cyclohexyl und 3-Methylcyclopentyl.

Die Erfindung betrifft ferner Verfahren zur Herstellung dieser Sulfonylharnstoffe sowie pharmazeutische Präparate, die diese enthalten oder aus ihnen bestehen.

Die Verfahren zur Herstellung sind dadurch gekennzeichnet, daß man

a) mit der Gruppe

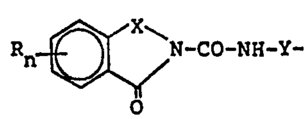

in 4-Stellung substituierte Benzolsulfonyl-isocyanate, -carbaminsäureester, -thiolcarbaminsäureester, -harnstoffe, -semicarbazide oder -semicarbazone mit einem Amin $R^1—NH_2$ oder dessen Salzen umsetzt oder Sulfonamide der Formel

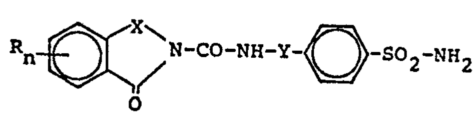

oder deren Salze mit $R^1$-substituierten Isocyanaten, Carbaminsäureestern, Thiolcarbaminsäureestern, Carbaminsäurehalogeniden oder Harnstoffen umsetzt,

b) mit der Gruppe

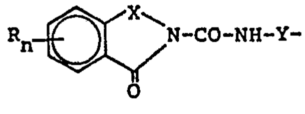

substituierte Benzolsulfonyl-isoharnstoffäther, -isothioharnstoffäther, -parabansäuren oder -halogenameisensäureamidine oder mit der Gruppe

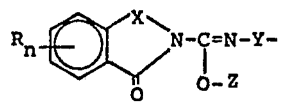

worin Z Methyl oder Aethyl bedeutet, substituierte Benzolsulfonylharnstoff spaltet,
c) in

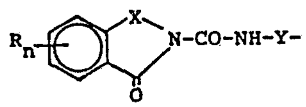

substituierten Benzolsulfonylthioharnstoffen das Schwefelatom durch Sauerstoff ersetzt,
d) entsprechende Benzolsulfinyl- oder -sulfenyl-harnstoffe oxydiert,
e) in Benzolsulfonylharnstoffen der Formel

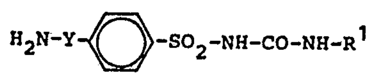

gegebenenfalls stufenweise den Rest

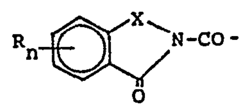

f) entsprechend substituierte Benzolsulfonylhalogenide mit $R^1$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N—$R^1$—N′-hydroxy-harnstoff umsetzt und die Reaktionsprodukte gegebenenfalls zur Salzbildung mit alkalischen Mitteln behandelt.

Die erwähnten Benzolsulfonyl-carbaminsäureester bzw. -thiolcarbaminsäureester können in der Alkoholkomponente einen Alkylrest oder einen Arylrest oder auch einen heterocyclischen Rest aufweisen. Da dieser Rest bei der Reaktion abgespalten wird, hat seine chemische Konstitution keinen Einfluß auf den Charakter des Endproduktes und kann deshalb in weiten Grenzen variiert werden. Das gleiche gilt für die N—$R^1$-substituierten Carbaminsäureester bzw. die entsprechenden Thiolcarbaminsäureester.

Als Carbaminsäurehalogenide eignen sich in erster Linie die Chloride.

Die als Ausgangsstoffe des Verfahrens infrage kommenden Benzolsulfonylharnstoffe können an der der Sulfonylgruppe abgewandten Seite des Harnstoffmoleküls unsubstituiert oder ein- oder insbesondere zweifach substituiert sein. Da diese Substituenten bei der Reaktion mit Aminen abgespalten werden, kann ihr Charakter in weiten Grenzen variiert werden. Neben alkyl-, aryl-, acyl- oder heterocyclisch substituierten Benzolsulfonylharnstoffen kann man auch Bensolsulfonylcarbamoylimidazole und ähnliche Verbindungen oder Bisbenzolsulfonylharnstoffe, die an einem der Stickstoffatome noch einen weiteren Substituenten z.B. Methyl, tragen können, verwenden. Man kann beispielsweise derartige Bis-(benzolsulfonyl)-harnstoffe oder auch N-Benzolsulfonyl-N′-acylharnstoffe mit $R^1$-substituierten Aminen behandeln und die erhaltenen Salze auf erhöhte Temperaturen, insbesondere solche oberhalb 100°C, erhitzen.

Weiterhin ist es möglich, von $R^1$-substituierten Harnstoffen auszugehen oder von solchen $R^1$-substituierten Harnstoffen, die am freien Stickstoffatom noch ein- oder insbesondere zweifach substituiert sind und diese mit

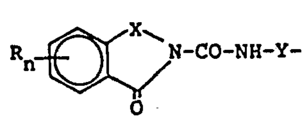

in 4-Stellung substituierten Benzolsulfonamiden umzusetzen. Als solche Ausgangsstoffe kommen beispielsweise infrage N-Cyclohexyl-harnstoff, die entsprechenden N′-Acetyl, N′-Nitro, N′-Cyclohexyl, N′,N′-Diphenyl- (wobei die beiden Phenylreste auch substituiert sowie direkt oder auch über ein Brückenglied wie —$CH_2$—, —NH—, —O— oder —S— miteinander verbunden sein können), N′-

Methyl-N'-phenyl-, N',N'-Dicyclohexylharnstoffe sowie Cyclohexyl-carbamoyl-imidazole, -pyrazole oder -triazole sowie solche der genannten Verbindungen, die anstelle des Cyclohexyls einen anderen im Bereich der Definition für $R^1$ liegenden Substituenten tragen.

Die Spaltung der als Ausgangsstoffe in Verfahren b) genannten Benzolsulfonylparabansäuren, -isoharnstoffäther, -isothioharnstoffäther oder -halogenameisensäureamidine sowie der genannten Benzolsulfonylharnstoffe erfolgt zweckmäßig durch alkalische Hydrolyse. Isoharnstoffäther können auch in einem sauren Medium mit gutem Erfolg gespalten werden.

Der Ersatz des Schwefelatoms in der Harnstoffgruppierung von entsprechend substituierten Benzolsulfonylthioharnstoffen durch ein Sauerstoffatom kann in bekannter Weise zum Beispiel mit Hilfe von Oxyden oder Salzen von Schwermetallen oder auch durch Anwendung von Oxydationsmitteln, wie Wasserstoffperoxid, Natriumperoxid, salpetriger Säure oder Permanganaten ausgeführt werden. Die Thioharnstoffe können auch entschwefelt werden durch Behandlung mit Phosgen oder Phosphorpentachlorid. Als Zwischenstufe erhaltene Chlorameisensäureamidine bzw. Carbodiimide können durch geeignete Maßnahmen wie Verseifen oder Anlagerung von Wasser in die Benzolsulfonylharnstoffe überführt werden.

Die Oxydation von Benzolsulfinyl- bzw. Benzolsulfenylharnstoffen erfolgt nach an sich bekannter Methode, vorzugsweise mit Oxydationsmitteln wie Permanganat oder Wasserstoffperoxid.

Die Acylierung der Sulfonylharnstoffe gemäß Verfahren e) kann mit reaktiven Derivaten der Säure

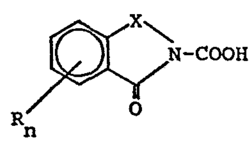

wie beispielsweise Halogeniden, erfolgen.

Als Sulfonyl- bzw. Sulfinylhalogenide gemäß Verfahren f) eignen sich insbesondere die Chloride. Als saures Kondensationsmittel kann man beispielsweise Thionylchlorid oder Polyphosphorsäure einsetzen.

Die Herstellung der physiologisch verträglichen Salze erfolgt nach an sich bekannten Methoden. Zur Salzbildung sind insbesondere geeignet Alkali- und Erdalkalihydroxyde, -carbonate oder -bicarbonate sowie physiologisch verträgliche organische Basen.

Die Ausführungsformen des Verfahrens gemäß der Erfindung können im allgemeinen hinsichtlich der Reaktionsbedingungen weitgehend variiert und den jeweiligen Verhältnissen angepaßt werden. Beispielsweise können die Umsetzungen in Abwesenheit oder Anwesenheit von Lösungsmitteln, bei Raumtemperatur oder bei erhöhter Temperatur durchgeführt werden.

Je nach dem Charakter der Ausgangsstoffe kann das eine oder andere der beschriebenen Verfahren in einzelnen Fällen einen gewünschten individuellen Benzolsulfonylharnstoff nur in geringen Ausbeuten liefern oder zu dessen Synthese nicht geeignet sein. In solchen verhältnismäßig selten auftretenden Fällen macht es dem Fachmann keine Schwierigkeiten, das gewünschte Produkt auf einem anderen der beschriebenen Verfahrenswege zu synthetisieren.

Die erhaltenen Verbindungen können durch Umfällen und/oder Umkristallisieren gereinigt werden. Die Reinigung kann auch erfolgen, indem man die Substanz aus einem kristallinen (Alkali)-Salz in einem geeigneten Lösungsmittel in Freiheit setzt.

Die erfindungsgemäßen Verbindungen zeichnen sich durch wertvolle pharmakologische Eigenschaften, insbesondere blutzuckersenkende, aus. Sie eignem sich daher als Arzneimittel, insbesondere als Antidiabetika.

Die blutzuckersenkende Wirkung der beschriebenen Benzolsulfonylharnstoffe kann dadurch festgestellt werden, daß man sie als freie Verbindungen oder in Form der Natriumsalze an normal ernährte Kaninchen verfüttert und den Blutzuckerwert nach der bekannten Methode von Hagedorn-Jensen oder mit einem Autoanalyzer über eine längere Zeitdauer ermittelt.

Die routinemäßige Bestimmung der blutzuckersenkenden Wirkung kann mit Dosierungen von z.B. 10,2 oder 0,4 mg Wirksubstanz pro kg Versuchstier nach bekannten Methoden erfolgen. Die folgenden Verbindungen I bis IV wurden in Dosierungen von 0,4 mg/kg Kaninchen verabreicht und die Blutzuckerwerte wurden mit einem Autoanalyzer über eine längere Zeitdauer ermittelt. Die hierbei gemessene Blutzuckersenkung ist in der nachfolgenden Tabelle in % nach Stunden angegeben.

I N - (4 - ⟨2 - (1 - Oxo - isoindolin - 2 - carboxamido) - äthyl⟩ - benzolsulfonyl) - N' - butyl - harnstoff

II N - (4 - ⟨2 - (1 - Oxo - isoindolin - 2 - carboxamido) - äthyl⟩ - benzolsulfonyl) - N' - cyclohexyl - harnstoff

III N - (4 - ⟨2 - (1 - Oxo - isoindolin - 2 - carboxamido) - äthyl⟩ - benzolsulfonyl) - N' - 3 - methyl - cyclopentyl - harnstoff

IV N - (4 - ⟨2 - (1 - Oxo - isoindolin - 2 - carboxamido) - äthyl⟩ - benzolsulfonyl) - N' - 2,5 - endomethylen - cyclohex - 3 - enyl - methyl - harnstoff

Tabelle

| Verbindung | Blutzuckersenkung am Kaninchen nach Verabreichung von 0,4 mg/kg p.o. in % nach Stunden | | | | | | | | |
|---|---|---|---|---|---|---|---|---|---|
| | 1 | 3 | 6 | 24 | 48 | 72 | 96 | 120 | 144 |
| I | 6 | 14 | 20 | 28 | 8 | 0 | | | |
| II | 33 | 35 | 47 | 48 | 54 | 40 | 0 | | |
| III | 20 | 27 | 44 | 50 | 68 | 64 | 60 | 32 | 0 |
| IV | 21 | 32 | 28 | 34 | 0 | | | | |

Die erfindungsgemäßen Acylureidoalkylbenzolsulfonylharnstoffe zeichnen sich durch eine starke und langanhaltende blutzuckersenkende Wirkung aus. Außerdem sind die Verbindungen gut verträglich.

Die Eigenschaften der Verbindungen erlauben es. in der Therapie des Diabetes mellitus mit so geringen Dosen auszukommen, daß da Präparat nur die verminderte Ansprechbarkeit des Pankreas auf einen erhöhten Blutzuckerspiegel wieder normalisiert.

Benzolsulfonylharnstoffe mit Ureidoalkylrest sind schon mehrfach beschrieben worden (DE—PS 14 43 911, DE—AS 16 70 700, DE—PS 16 18 389, DE—AS 22 38 870). Ferner wurden auch schon Acylaminoalkylbenzolsulfonylharnstoffe beschrieben, wobei der Acylaminoalkylrest die Oxoisoindolinoalkylgruppe darstellt (DE—OS 1 445 774 und DE—OS 1 670 660). Benzol sulfonylharnstoffe mit Acylureidoalkylrest sind noch nicht bekannt und es war nicht zur erwarten, daß sie sich durch die oben erwähnten günstigen Eigenschaften auszeichnen.

Die beschriebenen Sulfonylharnstoffe sollen vorzugsweise zur Herstellung von oral verabreichbaren Präparaten zur Behandlung des Diabetes mellitus dienen. Sie können als solche oder in Form ihrer Salze bzw. in Gegenwart von Stoffen, die zu einer Salzbildung führen, appliziert werden. Zur Salzbildung können beispielsweise alkalische Mittel wie Alkali- oder Erdalkalihydroxyde, -carbonate oder -bicarbonate herangezogen werden. Die Präparate können neben dem Sulfonylharnstoff bzw. dessen Salz auch noch andere Wirkstoffe enthalten.

Als medizinische Präparate kommen vorzugsweise Tabletten in Betracht, die neben den Verfahrenserzeugnissen die üblichen Träger- und Hilsstoffe wie Talkum, Stärke, Milchzucker oder Magnesiumstearat enthalten. Dabei kann es zweckmäßig sein, den oder die Wirkstoffe in gemahlener oder fein gefällter Form einzusetzen. Ein Präparat, das die beschrieben Benzolsulfonylharnstoffe als Wirkstoff enthält, z.B. eine Tablette oder ein Pulver mit oder ohne Zusätze, ist zweckmäßig in eine geeignet dosierte Form gebracht. Als Dosis ist dabei eine solche zu wählen, die der Wirksamkeit des verwendeten Benzolsulfonylharnstoffs und dem gewünschten Effekt angepaßt ist. Zweckmäßig beträgt die Dosierung je Einheit etwa 0,1 bis 10 mg, vorzugsweise 0,5 bis 2 mg, jedoch können auch darüber oder darunter liegende Dosierungseinheiten verwendet werden, die gegebenenfalls vor Applikation zu teilen bzw. zu vervielfachen sind.

Die nachfolgenden Beispiele zeigen einige der zahlreichen Verfahrensvarianten, die zur Synthese der erfindungsgemäßen Sulfonylharnstoffe verwendet werden können. Sie sollen jedoch nicht eine Einschränkung des Erfindungsgegenstandes darstellen.

Beispiel 1

N-(4-⟨2-(1-Oxo-isoindolin-2-carboxamido)-äthyl⟩-benzolsulfonyl)-N'-cyclohexyl-harnstoff

7,2 g 4-(2-⟨1-Oxo-isoindolin-2-carboxamido⟩-äthyl)-benzolsulfonamid (Schmp. 236—238°C, hergestellt durch Umsetzung von 1-Oxo-isoindolin-2-(N-2-phenyl-äthyl)-carboxamid ⟨Schmp. 146—148°C, hergestellt aus 1-Oxo-isoindolin und Phenyläthylisocyanat⟩ mit Chlorsulfonsäure und Reaktion des erhaltenen Sulfochlorids mit Ammoniak) werden in 300 ml Aceton und 5,5 g gemahlener Pottasche mehrere Stunden unter Rühren zum Sieden erhitzt. Anschließend gibt man 2,5 g Cyclohexylisocyanat zu und rührt 5 Stunden bei Siedetemperatur nach. Nach Kühlung saugt man das Kaliumsalz des gebildeten Sulfonylharnstoffs ab, löst in Wasser, filtriert und säuert das Filtrat mit verdünnter Salzsäure an. Der ausgefallene N-(4-⟨2-(1-Oxo-isoindolin-2-carboxamido)-äthyl⟩-benzol-sulfonyl)-N'-cyclohexyl-harnstoff wird abgesaugt und aus verdünntem Aceton umkristallisiert. Das Produkt schmilzt bei 214—216°C.

In analoger Weise erhält man den

N-(4-⟨2-(1-Oxo-isoindolin-2-carboxamido)-äthyl⟩-benzolsulfonyl)-N'-butyl-harnstoff vom Schmp. 188—190°C (aus verd. Aceton)

N-(4-⟨2-(1-Oxo-isoindolin-2-carboxamido)-äthyl⟩-benzolsulfonyl)-N'-hexyl-harnstoff vom Schmp. 179—181°C (aus Dioxan)

N-(4-<2-(1-Oxo-isoindolin-2-carboxamido)-äthyl>-benzolsulfonyl)-N'-äthyl-harnstoff vom Schmp.

209—211°C (aus verd. Aceton)

N-(4-⟨2-(1-Oxo-isoindolin-2-carboxamido)-äthyl⟩-benzolsulfonyl)-N'-4-methyl-cyclohexyl-harnstoff vom Schmp. 228—230°C (aus verd. Aceton)

N-(4-⟨2-(1-Oxo-isoindolin-2-carboxamido)-äthyl⟩-benzolsulfonyl)-N'-cyclohex-3-enyl-harnstoff vom Schmp. 199—201°C (aus wäßr. Aceton)

N - (4 - ⟨2 - (1 - Oxo - isoindolin - 2 - carboxamido) - äthyl⟩ - benzolsulfonyl) - N' - 2,5 - endomethylen - cyclohexyl - harnstoff von Schmp. 203—205°C (aus Dioxan)

N - (4 - ⟨2 - (1 - Oxo - isoindolin - 2 - carboxamido) - äthyl⟩ - benzolsulfonyl) - N' - 4,4 - dimethyl - cyclohexyl - harnstoff vom Schmp. 213—215°C (aus verd. Aceton)

N - (4 - ⟨2 - (1 - Oxo - isoindolin - 2 - carboxamido) - äthyl⟩ - benzolsulfonyl) - N' - cyclooctyl - harnstoff vom Schmp. 178—180°C (aus verd. Aceton)

N - (4 - ⟨2 - (1 - Oxo - isoindolin - 2 - carboxamido) - äthyl⟩ - benzolsulfonyl) - N' - benzyl - harnstoff von Schmp. 204—206°C (aus Dioxan)

N - (4 - ⟨2 - (1 - Oxo - isoindolin - 2 - carboxamido) - äthyl⟩ - benzolsulfonyl) - N' - 2,5 - endoäthylen - cyclohexyl - harnstoff

N - (4 - <2 - (1 - Oxo - isoindolin - 2 - carboxamido) - äthyl> - benzolsulfonyl) - N' - 2,5 - endomethylen - cyclohex - 3 - enyl - harnstoff

N - (4 - ⟨2 - (1 - Oxo - isoindolin - 2 - carboxamido) - äthyl⟩ - benzolsulfonyl) - N' - (4 - isopropyl - cyclohexyl) - harnstoff vom Schmp. 213—215°C (aus verdünntem Aceton)

N - (4 - ⟨2 - (1 - Oxo - isoindolin - 2 - carboxamido) - äthyl⟩ - benzolsulfonyl) - N' - cyclohex - 3 - enylmethyl - harnstoff vom Schmp. 203—205°C (aus verdünntem Aceton)

N - (4 - ⟨2 - (1 - Oxo - isoindolin - 2 - carboxamido) - äthyl⟩ - benzolsulfonyl) - N' - isobutyl - harnstoff vom Schmp. 196—198°C (aus verdünntem Aceton)

N - (4 - ⟨2 - (1 - Oxo - isoindolin - 2 - carboxamido) - äthyl⟩ - benzolsulfonyl) - N' - propyl - harnstoff vom Schmp. 200—202°C (aus verdünntem Aceton)

N - (4 - ⟨2 - (1 - Oxo - isoindolin - 2 - carboxamido) - äthyl⟩ - benzolsulfonyl) - N' - (2,5 - endomethylen - cyclohexyl - methyl) -harnstoff von Schmp. 203—205°C (aus verdünntem Dioxan)

N - (4 - ⟨2 - (1 - Oxo - isoindolin - 2 - carboxamido) - äthyl⟩ - benzolsulfonyl) - N' - (4 - methyl - cyclohex - 3 - enyl) - harnstoff vom Schmp. 211—213°C (aus Dioxan).

In analoger Weise erhält man aus dem 4-(2-⟨5-Methyl-1-oxo-isoindolin-2-carboxamido)-äthyl⟩-benzolsulfonamid (Schmp. 243—246°C, hergestellt aus 5-Methyl-1-oxo-isoindolin-2-(N-2-phenyläthyl)-carboxamid ⟨Schmp. 159—162°C, hergestellt aus 5-Methyl-1-oxo-isoindolin und Phenyläthylisocyanat⟩ und Chlorsulfonsäure und Reaktion des Sulfochlorids mit Ammoniak) den

N - (4 - ⟨2 - (5 - Methyl - 1 - oxo - isoindolin - 2 - carboxamido) - äthyl⟩ - benzolsulfonyl) - N' - cyclohexyl - harnstoff vom Schmp. 216—218°C (aus verdünntem Dioxan)

In analoger Weise erhält man aus dem 4-(2-⟨5-Chlor-1-oxo-isoindolin-2-carboxamido)-äthyl⟩-benzolsulfonamid (Schmp. 245—247°C, hergestellt aus 5-Chlor-1-oxo-isoindolin-2-(N-2-phenyläthyl)-carboxamid ⟨Schmp. 160—162°C, hergestellt aus 5-Chlor-1-oxo-isoindolin und Phenyläthylisocyanat⟩ und Chlorsulfonsäure und Reaktion des Sulfochlorids mit Ammoniak) den

N - (4 - ⟨2 - (5 - Chlor - 1 - oxo - isoindolin - 2 - carboxamido) - äthyl⟩ - benzolsulfonyl) - N' - cyclohexyl - harnstoff vom Schmp. 237—239°C (aus Aceton/Dioxan)

N - (4 - ⟨2 - (5 - Chlor - 1 - oxo - isoindolin - 2 - carboxamido) - äthyl⟩ - benzolsulfonyl) - N' - butyl - harnstoff vom Schmp. 215—217°C (aus verdünntem Dioxan)

In analoger Weise erhält man aus dem 4-(2-⟨1-Oxo-isoindolin-2-carboxamido⟩-propyl)-benzolsulfonamid (Schmp. 183—185°C, hergestellt durch Umsetzung von 1-Oxo-isoindolin-2-(N-2-phenylpropyl)-carboxamid Schmp. 126—128°C, hergestellt aus 1-Oxo-isoindolin und 2-Phenylpropyl-isocyanat mit Chlorsulfonsäure und Reaktion des erhaltenen Sulfochlorids mit Ammoniak) den

N - (4 - ⟨2 - (1 - Oxo - isoindolin - 2 - carboxamido) - propyl⟩ - benzolsulfonyl) - N' - cyclohexyl - harnstoff von Schmp. 197—198°C (aus Äthanol)

In analoger Weise erhält man aus dem 4-(2-⟨6-Methyl-1-oxo-isoindolin-2-carboxamido)-äthyl⟩-benzolsulfonamid (Schmp. 227—230°C, hergestellt aus 6-Methyl-1-oxo-isoindolin-2-(N-2-phenyläthyl)-carboxamid ⟨Schmp. 143—144°C, hergestellt aus 6-Methyl-1-oxoisoindolin und Phenyläthylisocyanat⟩ und Chlorsulfonsäure und Reaktion des Sulfochlorids mit Ammoniak) den

N - (4 - ⟨2 - (6 - Methyl - 1 - oxo - isoindolin - 2 - carboxamido) - äthyl⟩ - benzolsulfonyl) - N' - cyclohexyl - harnstoff vom Schmp. 212—214°C (aus verdünntem Dioxan)

N - (4 - ⟨2 - Methyl - 1 - oxo - isoindolin - 2 - carboxamido) - äthyl⟩ - benzolsulfonyl) - N' - butyl - harnstoff vom Schmp. 169—171°C (aus verd. Dioxan)

In analoger Weise erhält man aus dem 4-(2-⟨6-Chlor-1-oxo-isoindolin-2-carboxamido)-äthyl⟩-benzolsulfonamid (Schmp. 223—226°C, hergestellt aus 6-Chlor-1-oxo-isoindolin-2-(N-2-phenyläthyl)-carboxamid ⟨Schmp. 169—171°C, hergestellt aus 6-Chlor-1-oxo-isoindolin und Phenyläthylisocyanat⟩ und Chlorsulfonsäure und Reaktion des Sulfochlorids mit Ammoniak) den
N - (4 - ⟨2 - (6 - Chlor - 1 - oxo - isoindolin - 2 - carboxamido) - äthyl⟩ - benzolsulfonyl) - N' - cyclohexyl - harnstoff vom Schmp. 230—232°C (aus verd. Dioxan)
N - (4 - ⟨2 - (6 - Chlor - 1 - oxo - isoindolin - 2 - carboxamido) - äthyl⟩ - benzolsulfonyl) - N' - butyl - harnstoff vom Schmp. 208—210°C (aus verd. Dioxan)

In analoger Weise erhält man aus dem 4-(2-⟨4,6-Dichlor-1-oxo-isoindolin-2-carboxamido)-äthyl⟩-benzolsulfonamid (Schmp. 204—206°C, hergestellt aus 4,6-Dichlor-1-oxo-isoindolin-2-(N-2-phenyläthyl)-carboxamid ⟨Schmp. 137—139°C, hergestellt aus 4,6-Dichlor-1-oxo-isoindolin [Schmp. 282—284°C] und Phenyläthylisocyanat⟩ und Chlorsulfonsäure und Reaktion des Sulfochlorids mit Ammoniak) den
N - (4 - ⟨2 - (4,6 - Dichlor - 1 - oxo - isoindolin - 2 - carboxamido) - äthyl⟩ - benzolsulfonyl) - N' - cyclohexyl - harnstoff vom Schmp. 249—251°C (aus Dioxan)
N - (4 - ⟨2 - (4,6 - Dichlor - 1 - oxo - isoindolin - 2 - carboxamido) - äthyl⟩ - benzolsulfonyl) - N' - butyl - harnstoff vom Schmp. 190—192°C (aus verd. Dioxan)

In analoger Weise erhält man aus dem 4-(2-⟨3-Methyl-1-oxo-isoindolin-2-carboxamido)-äthyl⟩-benzolsulfonamid (Schmp. 193—195°C, hergestellt aus 3-Methyl-1-oxo-isoindolin-2-(N-phenyläthyl)-carboxamid ⟨Schmp. 80—82°C, hergestellt aus 3-Methyl-1-oxo-isoindolin und Phenyläthylisocyanat⟩ und Chlorsulfonsäure und Reaktion des Sulfochlorids mit Ammoniak) den
N - (4 - ⟨2 - (3 - Methyl - 1 - oxo - isoindolin - 2 - carboxamido) - äthyl⟩ - benzolsulfonyl) - N' - cyclohexyl - harnstoff vom Schmp. 124—125°C (aus Essigester)
N - (4 - ⟨2 - (3 - Methyl - 1 - oxo - isoindolin - 2 - carboxamido) - äthyl⟩ - benzolsulfonyl) - N' - butyl - harnstoff vom Schmp. 164—165°C (aus Äthanol-Essigester)
N - (4 - ⟨2 - (3 - Methyl - 1 - oxo - isoindolin - 2 - carboxamido) - äthyl⟩ - benzolsulfonyl) - N' - (4 - methyl - cyclohexyl) - harnstoff vom Schmp. 212—214°C (aus Äthanol-Dimethylformamid)

Beispiel 2

N - (4 - ⟨2 - (1 - Oxo - 1,2,3,4 - tetrahydro - isochinolin - 2 - carboxamido) - äthyl⟩ - benzolsulfonyl) - N' - cyclohexyl - harnstoff

5,5 g 4-(2-⟨1-Oxo-1,2,3,4-tetrahydroisochinolin-2-carboxamido⟩-äthyl)-benzolsulfonamid (Schmp. 197—198°C, hergestellt aus 1-Oxo-1,2,3,4-tetrahydroisochinolin2-(N-2-phenyl-äthyl)-carboxamid ⟨Schmp. 98—99°C, hergestellt aus 1-Oxo-1,2,3,4-tetrahydro-isochinolin und 2-Phenäthylisocyanat⟩ mit Chlorsulfonsäure und anschließende Umsetzung des Sulfochlorids mit Ammoniak) werden in 75 ml Aceton und 7,5 ml 2 n Natronlauge suspendiert und auf 0—5°C abgekühlt. Unter Rühren tropft man 2,15 g Cyclohexylisocyanat in 10 ml Aceton zu und rührt eine Stunde unter Eiskühlung und drei Stunden bei Raumtemperatur nach. Der Niederschlag wird durch Zugabe von Wasser in Lösung gebracht. Nach dem Entfernen des Acetons im Vakuum säuert man die verbleibende wäßrige Lösung mit verdünnter Salzsäure an. Der ausgefallene N-(4-⟨2-(1-Oxo-1,2,3,4-tetrahydro-isochinolin-2-carboxamido)-äthyl⟩-benzolsulfonyl)-N'-cyclohexyl-harnstoff wird abgesaugt, einmal aus verdünnter Ammoniaklösung mit verdünnter Salzsäure umgefällt und nach dem Absaugen und Trocknen aus Äthanol umkristallisiert. Das so erhaltene Produkt schmilzt bei 197—199°C.

In analoger Weise erhält man den
N - (4 - ⟨2 - (1 - Oxo - 1,2,3,4 - tetrahydro - isochinolin - 2 - carboxamido) - äthyl⟩ - benzolsulfonyl) - N' - 4 - methyl - cyclohexyl - harnstoff von Schmp. 203—205°C (aus Äthanol)
N - (4 - ⟨2 - (1 - Oxo - 1,2,3,4 - tetrahydro - isochinolin - 2 - carboxamido) - äthyl⟩ - benzolsulfonyl) - N' - butyl - harnstoff vom Schmp. 144—146°C (aus Äthanol)
N - (4 - ⟨2 - (1 - Oxo - 1,2,3,4 - tetrahydro - isochinolin - 2 - carboxamido) - äthyl⟩ - benzolsulfonyl) - N' - isobutyl - harnstoff vom Schmp. 167—168°C (aus Äthanol)
N - (4 - ⟨2 - (1 - Oxo - 1,2,3,4 - tetrahydro - isochinolin - 2 - carboxamido) - äthyl⟩ - benzolsulfonyl) - N' - 4 - äthyl - cyclohexyl - harnstoff vom Schmp. 168—170°C (aus Äthanol)

Beispiel 3

N - (4 - ⟨2 - (1 - Oxoo - isoindolin - 2 - carboxamido) - äthyl⟩ - benzolsulfonyl) - N' - cyclohexyl - harnstoff

4,2 g N - (4 - ⟨2 - Oxo - isoindolin - 2 - carboxamido) - äthyl⟩ - benzolsulfonyl) - carbamidsäuremethylester (Schmp. 215—217°C, hergestellt aus dem aus dem 4-(2-⟨1-Oxo-isoindolin-2-carboxamido⟩-äthyl)-benzolsulfonamid und Chlorameisensäuremethylester) werden in 100 ml Dioxan bei 50°C gelöst und mit 1 g Cyclohexylamin versetzt. Das ausgefallene Cyclohexylaminsalz des

Urethans geht bei einstündigem Kochen unter Rückfluß langsam in Lösung. Man engt auf 1/3 des Volumens ein und gießt die Restlösung auf verd. Salzsäure. Der in guter Ausbeute erhaltene N-(4-⟨2-(1-Oxo-isoindolin-2-carboxamido)-äthyl⟩-benzolsulfonyl)-N'-cyclohexyl-harnstoff wird abgesaugt und aus wäßrigem Aceton umkristallisiert. Er schmilzt bei 214—216°C.

In analoger Weise erhält man den
N - (4 - ⟨2 - (1 - Oxo - isoindolin - 2 - carboxamido) - äthyl⟩ - benzolsulfonyl) - N' - 2,5 - endomethylen - cyclohex - 3 - enyl - methyl - harnstoff vom Schmp. 204—206°C (aus wäßrigem Aceton)
N - (4 - ⟨2 - (1 - Oxo - isoindolin - 2 - carboxamido) - äthyl⟩ - benzolsulfonyl) - N' - cyclopentyl - harnstoff vom Schmp. 210—212°C (aus wäßrigem Aceton)
N - (4 - ⟨2 - (1 - Oxo - isoindolin - 2 - carboxamido) - äthyl⟩ - benzolsulfonyl) - N' - 3 - methyl - cyclopentyl - harnstoff vom Schmp. 194—196°C (aus wäßrigem Aceton)
N - (4 - ⟨2 - (1 - Oxo - isoindolin - 2 - carboxamido) - äthyl⟩ - benzolsulfonyl) - N' - 4 - isopropyl - cyclohexyl - harnstoff vom Schmp. 213—215°C (aus wäßrigem Aceton)
N - (4 - ⟨2 - (1 - Oxo - isoindolin - 2 - carboxamido) - äthyl⟩ - benzolsulfonyl) - N' - 4 - chlorcyclohexyl - harnstoff vom Schmp. 209—211°C (aus wässrigem Aceton)
N - (4 - ⟨2 - (1 - Oxo - isoindolin - 2 - carboxamido) - äthyl⟩ - benzolsulfonyl) - N' - cyclohex - 2 - enyl - methyl - harnstoff vom Schmp. 203—205°C (aus wäßrigem Aceton)
N - (4 - ⟨2 - (1 - Oxo - isoindolin - 2 - carboxamido) - äthyl⟩ - benzolsulfonyl) - N' - cyclopentylmethyl - harnstoff vom Schmp. 212—214°C (aus verdünntem Tetrahydrofuran)
N - (4 - ⟨2 - (1 - Oxo - isoindolin - 2 - carboxamido) - äthyl⟩ - benzolsulfonyl) - N' - (3 - äthyl - cyclopentyl) - harnstoff vom Schmp. 192—194°C (aus verd. Tetrahydrofuran)
N - (4 - ⟨2 - (1 - Oxo - isoindolin - 2 - carboxamido) - äthyl⟩ - benzolsulfonyl) - N' - nortricyclyl - harnstoff vom, Schmp. 226—228°C (aus Methanol/Dioxan)
N - (4 - ⟨2 - (1 - Oxo - isoindolin - 2 - carboxamido) - äthyl⟩ - benzolsulfonyl) - N' - sec - butyl - harnstoff vom Schmp. 187—189°C (aus Methanol)
N - (4 - ⟨2 - (1 - Oxo - isoindolin - 2 - carboxamido) - äthyl⟩ - benzolsulfonyl) - N' - (3,4 - dimethyl - cyclohexyl) - harnstoff vom Schmp. 201—203°C (aus verd. Methanol)
N - (4 - ⟨2 - (1 - Oxo - isoindolin - 2 - carboxamido) - äthyl⟩ - benzolsulfonyl) - N' - cyclohept - 2 - enyl - harnstoff vom Schmp. 196—198°C (aus Methanol-Dioxan)
N - (4 - ⟨2 - (1 - Oxo - isoindolin - 2 - carboxamido) - äthyl⟩ - benzolsulfonyl) - N' - (4 - methoxy - cyclohexyl) - harnstoff vom Schmp. 183—185°C (aus Methanol)
N - (4 - ⟨2 - (1 - Oxo - isoindolin - 2 - carboxamido) - äthyl⟩ - benzolsulfonyl) - N' - cyclopent - 2 - enyl - harnstoff vom Schmp. 196—198°C (aus Methanol-Dioxan)
N - (4 - ⟨2 - (1 - Oxo - isoindolin - 2 - carboxamido) - äthyl⟩ - benzolsulfonyl) - N' - cyclobutyl - harnstoff vom Schmp. 206—208°C (aus verd. Aceton)
N - (4 - ⟨2 - (1 - Oxo - isoindolin - 2 - carboxamido) - äthyl⟩ - benzolsulfonyl) - N' - (3 - methyl - cyclopentylmethyl) - harnstoff vom Schmp. 192—194°C (aus Methanol/Wasser).

## Beispiel 4

N - (4 - ⟨2 - (1 - Oxo - 1,2,3,4 - tetrahydro - isochinolin - 2 - carboxamido) - äthyl⟩ - benzolsulfonyl) - N' - 2,5 - endomethylen - cyclohexyl - harnstoff

2,08 g N-(4-⟨2-(1-Oxo-1,2,3,4-tetrahydro-isochinolin-2-carboxamido)-äthyl⟩-benzolsulfonyl)-harnstoff (Schmp. 200—201°C, hergestellt aus dem 4-(2-⟨1-Oxo-1,2,3,4-tetrahydroisochinolin-2-carboxamido)-äthyl⟩-benzolsulfonamid und Kaliumcyanat) werden in 25 ml Dioxan mit 1,47 g 2,5-Endomethylencyclohexylamin-Hydrochlorid und 0,5 g Triäthylamin eine Stunde unter Rückfluß gerührt. Nach dem Erkalten engt man im Vakuum ein, nimmt den Rückstand in Wasser auf, filtriert und säuert mit 2 n Salzsäure an. Der Niederschlag wird abgesaugt, aus verd. Ammoniak-Lösung mit verd. Salzsäure umgefällt und nach erneutem Absaugen aus Äthanol umkristallisiert. Der so erhaltene N - (4 - ⟨2 - (1 - Oxo - 1,2,3,4 - tetrahydro - isochinolin - 2 - carboxamido) - äthyl⟩ - benzolsulfonyl)-N' - 2,5 - endomethylen - cyclohexyl - harnstoff schmilzt bei 191—193°C.

## Beispiel 5

N - (4 - ⟨2 - (1 - Oxo - isoindolin - 2 - carboxamido) - äthyl⟩ - benzolsulfonyl) - N' - cyclohexyl-harnstoff

0,3 g N-(4-⟨2-(1-Oxo-isoindolin-2-carboxamido)-äthyl⟩-benzosulfonyl)-N'-cyclohexyl-thioharnstoff (Schmp. 194—196°C, hergestellt aus 4-⟨2-(1-Oxo-isoindolin-2-carboxamido)-äthyl⟩-benzolsulfonamid und Cyclohexylsenföl) werden in einem Gemisch aus 50 ml Wasser und 50 ml Methanol mit 0,3 g Quecksilberoxid versetzt und 3 Std. bei 40—45° gerührt. man saugt das abgeschiedene Quecksilbersulfid ab, engt das Filtrat unter vermindertem Druck ein, behandelt den Rückstand mit sehr verdünntem Ammoniak, filtriert und säuert das Filtrat mit verdünnter Salzsäure an. Der ausgefällte N-(4-⟨2-(1-Oxo-isoindolin-2-carboxamido)-äthyl⟩-benzolsulfonyl)-N'-cyclohexyl-harnstoff wird aus verd. Aceton umkristallisiert und schmilzt bei 211—213°C. Mischschmelzpunkt mit auf dem nach Beispiel 1 erhaltenen Produkt ohne Depression.

Beispiel 6

N - (4 - ⟨2 - (1 - Oxo - isoindolin - 2 - carboxamido) - äthyl⟩ - benzolsulfonyl) - N' - cyclohexyl-harnstoff

0,5 g N-(4-⟨2-(1-Oxo-isoindolin-2-carboxamido)-äthyl⟩-benzolsulfonyl)-N'-cyclohexyl-isoharnstoff-methyläther (Schmp. 180—182°C, hergestellt aus dem N-(4-⟨2-(1-Oxo-isoindolin-2-carboxamido)-äthyl⟩-benzolsulfonyl)-N'-cyclohexyl-thioharnstoff durch Entschwefelung mit Quecksilberoxid in Methanol bei 40°C) werden in 5 ml Dioxan mit 2 ml konz. Salzsäure einige Minuten auf dem Dampfbad erhitzt. Anschließend versetzt man mit Wasser und Eis und saugt das ausgefällte Produkt ab. Nach dem Umkristallisieren schmilzt der N-(4-⟨2-(1-Oxo-isoindolin-2-carboxamido)-äthyl⟩-benzolsulfonyl)-N'-cyclohexyl-harnstoff bei 214—216°C.

In analoger Weise erhält man aus dem

N - (4 - ⟨2 - (1 - Oxo - isoindolin - 2 - carboxamido) - äthyl⟩ - benzolsulfonyl) - N' - cyclohexyl - isothioharnstoffmethyläther (Schmp. 149—151°C, hergestellt aus dem N-(4-⟨2-(1-Oxo-isoindolin-2-carboxamido)-äthyl⟩-benzolsulfonyl)-N'-cyclohexylharnstoff und Methyljodid in Dioxan) durch kurzes Erhitzen mit Natronlauge in Dioxan auf 50° den N-(4-⟨2-(1-Oxo-isoindolin-2-carboxamido)-äthyl⟩-benzol-sulfonyl)-N'-cyclohexyl-harnstoff vom Schmp. 214—216°C nach Umkristallisieren aus verdünntem Aceton.

Beispiel 7

N - (4 - ⟨2 - (1 - Oxo - isoindolin - 2 - carboxamido) - äthyl⟩ - benzolsulfonyl) - N' - cyclohexyl - harnstoff

1 g 4-⟨2-(1-Oxo-isoindolin-2-carboxamido)-äthyl⟩-benzolsulfinsäurechlorid (Rohprodukt, hergestellt durch Reduktion des Sulfochlorids mit Natriumsulfit und Umsetzung der so erhaltenen Sulfinsäure mit Thionylchlorid) wird bei Raumtemperatur in eine Lösung von 0,7 g Cyclohexylharnstoff in 10 ml Pyridin eingetragen und 15 Min. gerührt. Anschließend gießt man da Reaktionsgemisch in Wasser, saugt das ausgefällte Produkt ab, behandelt mit verdünntem Ammonia und saugt wieder ab. Das Rohprodukt schmilzt bei 170—172°C. 0,3 g der erhaltenen Verbindung werden in Dimethylformamid gelöst und mit wäßriger Kaliumpermanganatlösung versetzt, bis die Permanganatfarbe bestehen bleibt. Der Permanganatüberschuß wird mit Natriumsulfit beseitigt, die Lösung filtriert und mit verdünnter Salzsäure angesäuert. Die ausgefällte Verbindung wird noch einmal aus sehr verdünntem Ammoniak umgefällt und aus verdünntem Aceton umkristallisiert.

Der N-(4-⟨2-(1-Oxo-isoindolin-2-carboxamido)-äthyl⟩-benzolsulfonyl)-N'-cyclohexyl-harnstoff schmilzt bei 212—214°C und gibt mit der nach Beispiel 1 erhaltenen Substanz keine Depression.

Beispiel 8

N - (4 - ⟨2 - (1 - Oxo - isoindolin - 2 - carboxamido) - äthyl⟩ - benzolsulfonyl) - N' - butyl - harnstoff

3,4 g 4-⟨2-(1-Oxo-isoindolin-2-carboxamido)-äthyl⟩-benzolsulfinsäure (Rohprodukt, hergestellt durch Reduktion des Sulfochlorids mit Natriumsulfit) und 1,4 g N-Hydroxy-N'-butyl-harnstoff werden in 40 ml Dioxan suspendiert und unter Rühren tropfenweise mit einer Lösung von 1 ml Thionylchlorid in 10 ml Dioxan versetzt. Anschließend erhitzt man 2 Std. auf 60°, dabei entsteht ein klare Lösung. Anschließend gießt man in Wasser, behandelt die ausgefällte Substanz mit verdünntem Ammoniak, filtriert, säuert an und kristallisiert aus verdünntem Aceton um. Der erhaltene N-(4-⟨2-(1-Oxo-isoindolin-2-carboxamido)-äthyl⟩-benzolsulfonyl)-N'-butyl-harnstoff schmilzt bei 188—190°C.

Beispiel 9

N - (4 - ⟨2 - (1 - Oxo - isoindolin - 2 - carboxamido) - äthyl⟩ - benzolsulfonyl) - N' - butyl - harnstoff

3 g N-(4-⟨2-Amino-äthyl⟩-benzolsulfonyl)-N'-butyl-harnstoff (Schmp. 210—212°C, hergestellt durch Verseifung von N-(4-⟨2-Acetylamino-äthyl⟩-benzolsulfonyl)-N'-butyl-harnstoff mit Natronlauge) werden mit 0,4 g Natriumhydroxid in 50 ml Wasser/50 ml Aceton gelöst und unter Rühren mit einer Lösung von 2 g 1-Oxo-isoindolin-2-carbonsäurechlorid (Schmp. 119—121°, hergestellt aus 1-Oxo-isoindolin-Natrium und Phosgen) in etwa 50 ml Aceton versetzt. Die Temperatur des Ansatzes steigt dabei etwas an. Man rührt 2 Std. bei Raumtemperatur nach und säuert den Rückstand mit verdünnter Salzsäure an. Die ausgefällte Substanz wird aus verdünntem Ammoniak umgefällt und aus verdünntem Aceton umkristallisiert. Der erhaltene N-(4-⟨2-(1-Oxo-isoindolin-2-carboxamido)-äthyl⟩-benzolsulfonyl)-N'-butyl-harnstoff schmilzt bei 188—190°C.

Beispiel 10

N - (4 - ⟨2 - (1 - Oxo - isoindolin - 2 - carboxamido) - äthyl⟩ - benzolsulfonyl) - N' - butyl - harnstoff

4,8 g Butylharnstoff werden in 100 ml Tetrahydrofuran gelöst, unter Rühren mit 1,5 g 80 prozentigem Natriumhydrid (in Öl) versetzt und 3 Std. auf 60°C erhitzt. Anschließend gibt man unter Kühlung 7,6 g 4-⟨2-(1-Oxo-isoindolin-2-carboxamido)-äthyl⟩-benzol-sulfochlorid in 100 ml

Tetrahydrofuran zu, steigert die Temperatur langsam auf 60—70°C und rührt bei dieser Temperatur 3 Stunden nach. Das Lösungsmittel wird unter vermindertem Druck abgezogen, der Rückstand mit Wasser versetzt, filtriert und das Filtrat mit verdünnter Salzsäure angesäuert. Nach Umkristallisieren aus verd. Aceton schmilzt der N-(4-⟨2-(1-Oxo-isoindolin-2-carboxamido)-äthyl⟩-benzolsulfonyl)-N'-butyl-harnstoff bei 189—190°C.

Beispiel 11

N - (4 - ⟨2 - (1 - Oxo - isoindolin - 2 - carboxamido) - äthyl⟩ - benzolsulfonyl) - N' - butyl - harnstoff

4 g N-(4-⟨2-(1-Oxo-isoindolin-2-carboxamido)-äthyl⟩-benzolsulfonyl)-harnstoff (Schmp. 207—209°C, hergestellt aus 4-⟨2-(1-Oxo-isoindolin-2-carboxamido)-äthyl⟩-benzolsulfonamid und Kaliumcyanat in 80 proz. Athanol) werden mit 0,73 g Butylamin in 100 ml Dioxan am Rückflußkühler 1 Stunde zum Sieden erhitzt. Anschließend destilliert man das Lösungsmittel unter vermindertem Druck ab, fällt den Rückstand aus sehr verdünntem Ammoniak um und kristallisiert aus verdünntem Aceton um. Der erhaltene N-(4-⟨2-(1-Oxo-isoindolin-2-carboxamido)-äthyl⟩-benzolsulfonyl)-N'-butyl-harnstoff schmilzt bei 188—190°C.

Beispiel 12

N - (4 - ⟨2 - (1 - Oxo - isoindolin - 2 - carboxamido) - äthyl⟩ - benzolsulfonyl) - N' - cyclohexyl - harnstoff

3,6 g 4-(2-⟨1-Oxo-isoindolin-2-carboxamido)-äthyl⟩-benzolsulfonamid werden mit 2,5 g Trichloracetylcyclohexylamid und 2,8 g Kaliumcarbonat gut gemischt und im Bad 1 Std. auf 160°C erhitzt. Nach dem Abkühlen behandelt man das Reaktionsgemisch mit Wasser und Salzsäure, saugt ab, fällt aus sehr verdünntem Ammoniak um und kristallisiert aus verdünntem Aceton um. Der erhaltene N-(4-⟨2-(1-Oxo-isoindolin-2-carboxamido)-äthyl⟩-benzolsulfonyl)-N'-cyclohexyl-harnstoff schmilzt bei 214—216°C.

**Patentansprüche für die Vertragsstaaten: BE CH DE FR GB IT LU NL SE**

1. Sulfonylharnstoffe der Formal

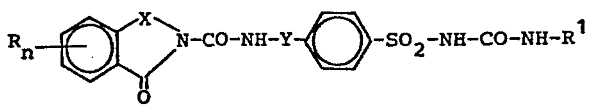

in welcher bedeuten:

n = 1 oder 2

R Wasserstoff, Alkyl mit 1 bis 4 Kohlenstoffatomen, Alkoxy mit 1 bis Kohlenstoffatomen oder Halogen, wobei, falls n 2 ist, die R gleich oder verschieden sein können

X eine $—CH_2—$, $—CH_2—CH_2—$ oder

$$\begin{matrix} CH_3 \\ | \\ —CH— \end{matrix} \text{ -Gruppe,}$$

Y Alkylen mit 2 oder 3 C-Atomen

$R^1$ Alkyl von 2 bis 8 C-Atomen, Cycloalkyl, Alkylcycloalkyl, Dialkylcycloalkyl, Cycloalkylalkyl, Cycloalkenyl, Alkylcycloalkenyl mit jeweils 4 bis 9 C-Atomen, Methylcyclopentylmethyl, Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl,

und deren physiologisch verträgliche Salze.

2. Verbindung gemäß Anspruch 1, dadurch gekennzeichnet, daß Y die $—CH_2—CH_2$-Gruppe bedeutet.

3. Verbindung gemäß Anspruch 1, dadurch gekennzeichnet, daß X die $—CH_2$-Gruppe bedeutet.

4. Verbindung gemäß Anspruch 1, dadurch gekennzeichnet, daß R Wasserstoff, Methyl oder Chlor bedeutet.

5. Verbindung gemäß Anspruch 1, dadurch gekennzeichnet, daß $R^1$ Butyl, Isobutyl, Methylcyclopentyl, Cyclohexyl oder Cyclopentylmethyl bedeutet.

6. Verbindung gemäß Anspruch 1, dadurch gekennzeichnet, daß Y die $—CH_2—CH_2$-Gruppe, X die $—CH_2$-Gruppe, R Wasserstoff und $R^1$ 3-Methylcyclopentyl oder Cyclohexyl bedeutet.

7. Verfahren zur Herstellung von Sulfonylharnstoffen gemäß Anspruch 1, dadurch gekennzeichnet, daß man

a) mit der Gruppe

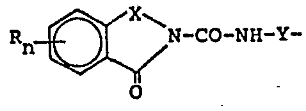

in 4-Stellung substituierte Benzolsulfonyl-isocyanate, -carbaminsäureester, -thiocarbaminsäureester, -harnstoffe, -semicarbazide oder -semicarbazone mit einem Amin $R^1$—$NH_2$ oder dessen Salzen umsetzt oder Sulfonamide der Formel

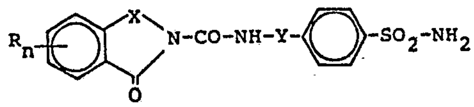

oder deren Salze mit $R^1$-substituierten Isocyanaten, Carbaminsäureestern, Thiolcarbaminsäureestern, Carbaminsäurehalogeniden oder Harnstoffen umsetzt,

b) mit der Gruppe

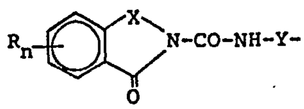

substituierte Benzolsulfonyl-isoharnstoffäther -isothioharnstoffäther, -parabansäuren oder -halogenameisensäureamidine oder mit der Gruppe

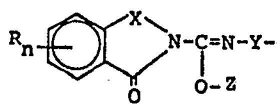

worin Z Methyl oder Aethyl bedeutet, substituierte Benzolsulfonylharnstoffe spaltet,

c) in

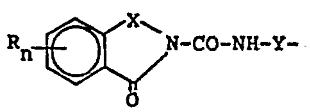

substituierten Benzolsulfonylthioharnstoffen das Schwefelatom durch Sauerstoff ersetzt,

d) entsprechende Benzolsulfinyl- oder -sulfenylharnstoffe oxydiert,

e) in Benzolsulfonylharnstoffen der Formel

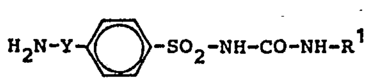

gegebenenfalls stufenweise den Rest

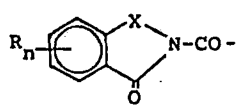

einführt,

f) entsprechend substituierte Benzolsulfonylhalogenide mit $R^1$-substituierten Harnstoffen oder deren Alkalisalzen umsetzt oder entsprechend substituierte Benzolsulfinsäure-halogenide oder in Gegenwart von sauren Kondensationsmitteln, auch entsprechend substituierte Sulfinsäuren oder deren Alkalisalze, mit N—$R^1$—N'-hydroxy-harnstoff umsetzt und die Reaktionsprodukte gegebenenfalls zur Salzbildung mit alkalischen Mitteln behandelt.

8. Arzneimittel enthaltend einen Sulfonylharnstoff gemäß Anspruch 1 oder eines seiner Salze.

**Claims for the Contracting States: BE CH DE FR GB IT LU NL SE**

1. Sulfonylureas of the formula

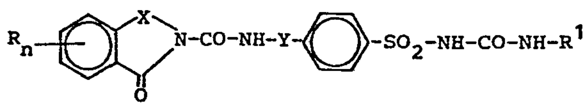

in which the symbols have the following meanings:

n: 1 or 2

R: hydrogen, alkyl with 1 to 4 carbon atoms, alkoxy with 1 to 4 carbon atoms or halogen which, with n being 2, may be identical or different,

X: a $—CH_2—$, $—CH_2—CH_2—$ or

$$—\underset{}{\overset{CH_3}{\overset{|}{CH}}}—$$ group

Y: alkylene with 2 or 3 C atoms,

$R^1$: alkyl of 2 to 8 C atoms, cycloalkyl, alkylcycloalkyl, dialkylcycloalkyl, cycloalkylalkyl, cycloalkenyl or alkylcycloalkenyl, in each case with 4—9 C atoms, methylcyclopentylmethyl, cyclohexenylmethyl, chlorocyclohexyl, methoxycyclohexyl, bicyclohepty, bicycloheptenyl, bicycloheptylmethyl, bicycloheptenylmethyl, bicyclooctyl, nortricyclyl, adamantyl or benzyl, and their physiologically tolerated salts.

2. The compound of claim 1, wherein Y denotes the $—CH_2—CH_2$-group.

3. The compound of claim 1, wherein X denotes the $—CH_2$-group.

4. The compound of claim 1, wherein R denotes hydrogen, methyl or chlorine.

5. The compound of claim 1, wherein $R^1$ denotes butyl, isobutyl, methylcyclopentyl, cyclohexyl or cyclopentylmethyl.

6. The compound of claim 1, wherein Y denotes the $—CH_2—CH_2$-group, X denotes the $CH_2$-group, R denotes hydrogen and $R^1$ denotes 3-methylcyclopentyl or cyclohexyl.

7. Process for the manufacture of sulfonylureas as claimed in claim 1, which comprises

a) reacting benzenesulfonyl-isocyanates, -carbamic acid esters, -thiolcarbamic acid esters, -ureas, -semicarbazides or -semicarbazones, which are substituted in the 4-position by the group

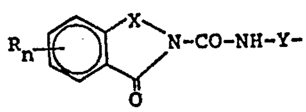

with an amine $R^1—NH_2$ or its salts, or sulfonamides of the formula

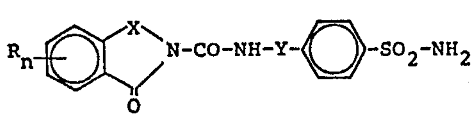

or their salts with $R^1$-substituted isocyanates, carbamic acid esters, thiolcarbamic acid esters, carbamic acid halides or ureas,

b) splitting benzenesulfonyl-isourea-ethers, -isothiourea-ethers, -parabanic acids or -halo formamidines substituted by the group

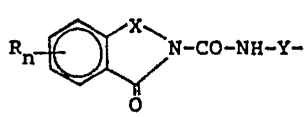

or benzenesulfonylureas substitued by the group

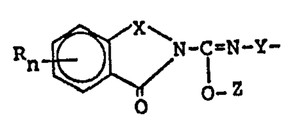

in which Z denotes methyl or ethyl are caused to undergo scission,

c) replacing in benzenesulfonylthioureas substituted by

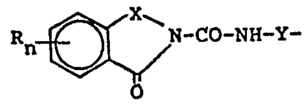

the sulfur atom by an oxygen atom,

d) oxidizing corresponding benzenesulfinyl-ureas or -sulfenylureas

e) introducing the radical

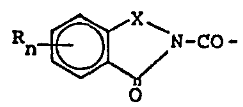

if appropriate stepwise, into benzenesulfonylureas of the formula

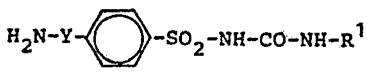

f) reacting correspondingly substituted benzenesulfonyl halides with $R^1$-substituted ureas of their alkali metal salts or correspondingly substituted benzenesulfinic acid halides or, reacting, in the presence of acid condensation agents, also correspondingly substituted sulfinic acids of their alkali metal salts, with N—$R^1$—N'-hydroxy-urea and, if desired, treating the reaction products with alkaline agents in order to form the salts.

8. Medicament containing a sulfonylurea according to claim 1, or one of its salts.

**Revendications pour les Etats contractants: BE CH DE FR GB IT LU NL SE**

1. Sulfonyl-urées de formule

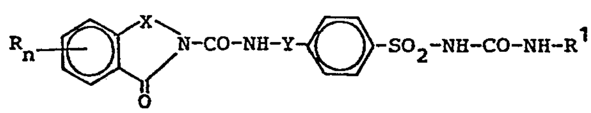

dans laquelle

n = 1 ou 2

R représente l'hydrogène, un radical alkyle ayant de 1 à 4 atomes de carbone, un radical alcoxy ayant de 1 à 4 atomes de carbone ou un halogène, les groupes R pouvant être identiques ou différents, lorsque n = 2

X représente un groupe $—CH_2—$, $CH_2—CH_2—$ ou

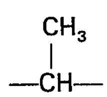

Y représente un radical alkylène ayant 2 ou 3 atomes de carbone,

$R^1$ représente un radical alkyle ayant de 2 à 8 atomes de carbone, un radical cycloalkyle, alkylcycloalkyle, dialkylcycloalkyle, cycloalkylalkyle, cycloalcényle, alkylcycloalcényl ayant chacun de 4 à 9 atomes de carbone, un radical méthylcyclopentyl-méthyle, cyclohexénylméthyle, chlorocyclohexyle, méthoxycyclohexyle, bicycloheptyle, bicycloheptényle, bicycloheptylméthyle, bicycloheptényIméthyle, bicyclooctyle, nortricyclyle, adamantyle ou benzyle,

et leurs sels physiologiquement compatibles.

2. Composé selon la revendication 1, caractérisé en ce que Y représente le groupe $—CH_2—CH_2—$.

3. Composé selon la revendication 1, caractérisé en ce que X représente le groupe $—CH_2—$.

4. Composé selon la revendication 1, caractérisé en ce que R représente l'hydrogène, le groupe méthyle ou le chlore.

5. Composé selon la revendication 1, caractérisé en ce que $R^1$ représente un groupe butyle, isobutyle, méthylcyclopentyle, cyclohexyle ou cyclopentylméthyle.

6. Composé selon la revendication 1, caractérisé en ce que Y représente le groupe $—CH_2—CH_2$, X représente le groupe $—CH_2—$, R représente l'hydrogène et $R^1$ représente le groupe 3-méthylcyclopentyle ou le groupe cyclohexyle.

7. Procédé de préparation de sulfonyl-urées selon la revendication 1, caractérisé en ce que a) on

fait réagir des benzène-sulfonyl-isocyanates, des esters de l'acide benzène-sulfonyl-carbamique, des esters de l'acide benzène-sulfonyl-thio-carbamique, des benzène-sulfonyl-urées, des benzènes-sulfonyl-semicarbazides ou des benzène-sulfonyl-semicarbazones substitués en position 4 par le groupe

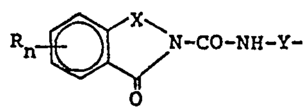

avec une amine $R^1$ —$NH_2$ ou ses sels, ou des sulfonamides de formule

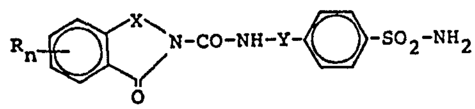

ou leurs sels avec des isocyanates, des esters de l'acide carbamique, des esters de l'acide thio-carbamique, des halogénures de l'acide carbamique ou des urées substitués par $R^1$, b) on coupe des éthers de benzène-sulfonyl-isourée, des éthers de benzène-sulfonyl-isothiourée, des acides benzène-sulfonyl-parabaniques ou des amidines de l'acide benzène-sulfonyl-halogéno-formique substitués par le groupe

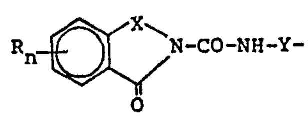

ou des benzène-sulfonyl-urées substituées par le groupe

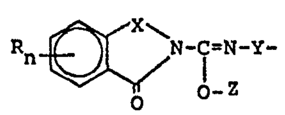

où Z représente un radical méthyle ou éthyle, c) dans les benzène-sulfonyl-thio urées substituées par

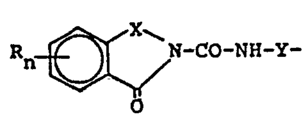

on remplace l'atome de soufre par l'oxygène, d) on oxyde des benzène-sulfinyl-urées ou des benzène-sulfényl-urées correspondantes, e) dans des benzène-sulfonyl-urées de formule

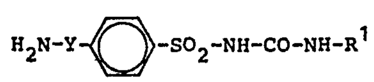

on introduit, éventuellement par étapes, le groupe

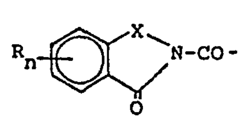

f) on fait réagir des benzènes sulfonyl-halogénures substitués de façon correspondante, avec des urées substituées par $R^1$ ou avec leurs sels alcalins, ou on fait réagir des halogénures de l'acide benzène sulfinique substitués de façon correspondante, ou, en présence d'agents de condensation acides, également des acides sulfiniques substitués de façon correspondante ou leurs sels alcalins, avec une N—$R^1$—N'-hydroxy-urée et éventuellement on traite les produits de réaction avec des agents alcalins pour former des sels.

8. Médicaments, caractérisés en ce qu'ils contiennent une sulfonyl-urée selon la revendication 1, ou un de ses sels.

**Patentanspruch für den Vertragsstaat: AT**

Verfahren zur Herstellung von Sulfonylharnstoffen der Formel

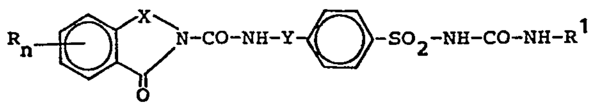

in welcher bedeuten:

n = 1 oder 2

R Wasserstoff, Alkyl mit 1 bis 4 Kohlenstoffatomen, Alkoxy mit 1 bis Kohlenstoffatomen oder Halogen, wobei, falls n 2 ist, die R gleich oder verschieden sein können

X eine $—CH_2—$, $—CH_2—CH_2—$ oder

$CH_3$
|
—CH— -Gruppe,

Y Alkylen mit 2 oder 3 C-Atomen

$R^1$ Alkyl von 2 bis 8 C-Atomen, Cycloalkyl, Alkylcycloalkyl, Dialkylcycloalkyl, Cycloalkylalkyl, Cycloalkenyl, Alkylcycloalkenyl mit jeweils 4 bis 9 C-Atomen, Methylcyclopentylmethyl, Cyclohexenylmethyl, Chlorcyclohexyl, Methoxycyclohexyl, Bicycloheptyl, Bicycloheptenyl, Bicycloheptylmethyl, Bicycloheptenylmethyl, Bicyclooctyl, Nortricyclyl, Adamantyl oder Benzyl,

und von deren physiologisch verträglichen Salzen, dadurch gekennzeichnet, daß man

a) mit der Gruppe

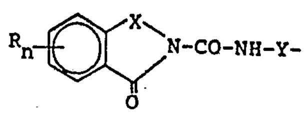

in 4-Stellung substituierte Benzolsulfonyl-isocyanate, -carbaminsäureester, -thiolcarbaminsäureester, -harnstoffe, -semicarbazide oder -semicarbazone mit einem Amin $R^1—NH_2$ oder dessen Salzen umsetzt oder Sulfonamide der Formel

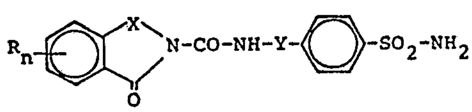

oder deren Salze mit $R^1$-substituierten Isocyanaten, Carbaminsäureestern, Thiolcarbaminsäureestern, Carbaminsäurehalogeniden oder Harnstoffen umsetzt,

b) mit der Gruppe

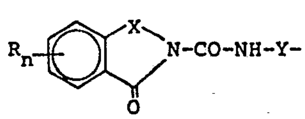

substituierte Benzolsulfonyl-isoharnstoffäther, -isothioharnstoffäther, -parabensäuren oder -halogenameisensäureamidine oder mit der Gruppe

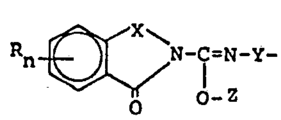

worin Z Methyl oder Aethyl bedeutet, substituierte Benzolsulfonylharnstoffe spaltet,

c) in

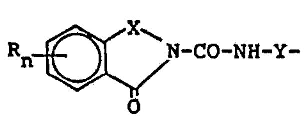

substituierten Benzolsulfonylthioharnstoffen das Schwefelatom durch Sauerstoff ersetzt,

d) entsprechende Benzolsulfinyl- oder -sulfenylharnstoffe oxydiert,

e) in Benzolsulfonylharnstoffen der Formel

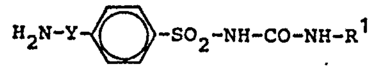

gegebenenfalls stufenweise den Rest

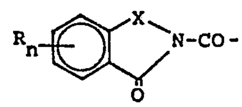

einführt,

f) entsprechend substituierte Benzolsulfonylhalogenide mit $R^1$-substituierten Harnstoffen oder deren Alkalisalzen umsetzt oder entsprechend substituierte Benzolsulfinsäure-halogenide oder in Gegenwart von sauren Kondensationsmitteln, auch entsprechend substituierte Sulfinsäuren oder deren Alkalisalze, mit $N—R^1—N'$-hydroxy-harnstoff umsetzt und die Reaktionsprodukte gegebenenfalls zur Salzbildung mit alkalischen Mitteln behandelt.

**Claim for the Contracting State: AT**

Process for the manufacture of sulfonylureas of the formula

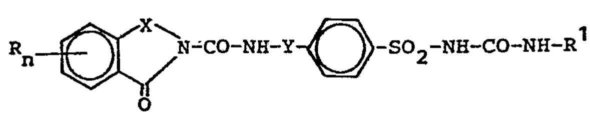

in which the symbols have the following meanings:

n: 1 or 2

R: hydrogen, alkyl with 1 to 4 carbon atoms, alkoxy with 1 to 4 carbon atoms or halogen which, with n being 2, may be identical or different,

X: a $—CH_2—$, $—CH_2—CH_2—$ or

$$—\overset{CH_3}{\overset{|}{CH}}—$$ group

Y: alkylene with 2 or 3 C atoms,

$R^1$: alkyl of 2 to 8 C atoms, cycloalkyl, alkylcycloalkyl, dialkylcycloalkyl, cycloalkylalkyl, cycloalkenyl or alkylcycloalkenyl, in each case with 4—9 C atoms, methylcyclopentylmethyl, cyclohexenylmethyl, chlorocyclohexyl, methoxycyclohexyl, bicyclohepty, bicycloheptenyl, bicycloheptylmethyl, bicycloheptenylmethyl, bicyclooctyl, nortricyclyl, adamantyl or benzyl, and of their physiologically tolerated salts which comprises

a) reacting benzenesulfonyl-isocyanates, -carbamic acid ester, -thiocarbamic acid esters, -ureas, -semicarbazides or -semicarbazones, which are substituted in the 4-position by the group

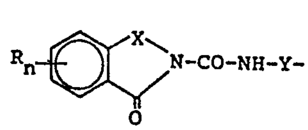

with an amine $R^1—NH_2$ or its salts, or sulfonamides of the formula

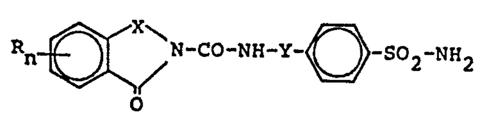

or their salts with $R^1$-substituted isocyanates, carbamic acid esters, thiolcarbamic acid esters, carbamic halides or ureas,

b) splitting benzenesulfonyl-isourea-ethers, -isothiourea-ethers, -parabanic acids or -haloformamidines substituted by the group

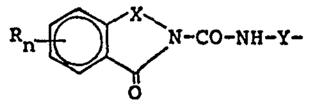

or benzenesulfonylureas substituted by the group

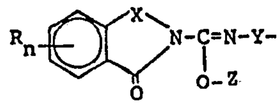

in which Z denotes methyl or ethyl are caused to undergo scission,

c) replacing in benzenesulfonylthioureas substituted by

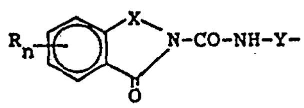

the sulfur atom by an oxygen atom,

d) oxidizing corresponding benzenesulfinyl-ureas or -sulfenylureas

e) introducing the radical

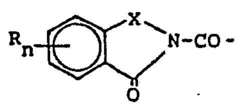

if appropriate stepwise, into benzenesulfonylureas of the formula

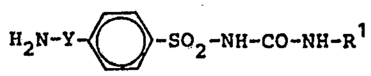

f) reacting correspondingly substituted benzenesulfonyl halides with $R^1$-substituted ureas or their alkali metal salts or correspondingly substituted benzenesulfinic acid halides or, reacting, in the presence of acid condensation agents, also correspondingly substituted sulfinic acids or their alkali metal salts, with N—$R^1$—N'-hydroxy-urea and, if desired, treating the reaction products with alkaline agents in order to form the salts.

**Revendication pour l'État contractant: AT**

Procédé de préparation de sulfonyl-urées de formule

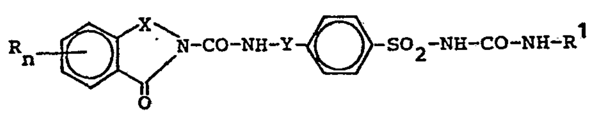

dans laquelle

n = 1 ou 2

R représente l'hydrogène, un radical alkyle ayant de 1 à 4 atomes de carbone, un radical alcoxy ayant de 1 à 4 atomes de carbone ou un halogène, les groupes R pouvant être identiques ou différents, lorsque n = 2

X représente un groupe —$CH_2$—, —$CH_2$—$CH_2$— ou

$CH_3$
|
—CH—

Y représente un radical alkylène ayant 2 ou 3 atomes de carbone,

$R^1$ représente un radical alkyle ayant de 2 à 8 atomes de carbone, un radical cycloalkyle, alkylcycloalkyle, dialkylcycloalkyle, cycloalkylalkyle, cycloalcényle, alkylcycloalcényle ayant chacun de 4 à 9 atomes de carbone, un radical méthylcyclopentyl-méthyle, cyclohexénylméthyle, chlorocyclohexyle, méthoxycyclohexyle, bicycloheptyle, bicycloheptényle, bicycloheptylméthyle, bicyclohepténylméthyle, bicyclooctyle, nortricyclyle, adamantyle ou benzyle,

et de leurs sels physiologiquement compatibles, caractérisé en ce que:

a) on fait réagir des benzène-sulfonyl-isocyanates, des esters de l'acide benzène-sulfonyl-carbamique, des esters de l'acide benzène-sulfonyl-thio-carbamique,des benzène-sulfonyl-urées, des benzène-sulfonyl-semicarbazides ou des benzène-sulfonyl-semicarbazones substitués en position 4 par le groupe

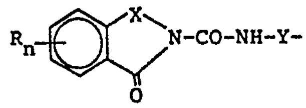

avec une amine $R^1$ —$NH_2$ ou ses sels, ou des sulfonamides de formule

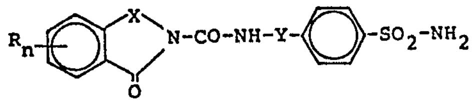

ou leurs sels avec des isocyanates, des esters de l'acide carbamique, des esters de l'acide thiocarbamique, des halogénures de l'acidse carbamique ou des urées substitués par $R^1$,

b) on coupe des éthers de benzène-sulfonyl-isourée, des éthers de benzène-sulfonyl-isothiourée, des acides benzène-sulfonyl-parabaniques ou des amidines de l'acide benzène-sulfonyl-halogénoformique substitués par le groupe

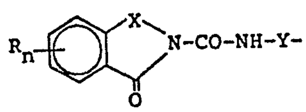

ou des benzène-sulfonyl-urées substituées par le groupe

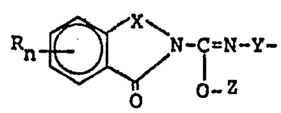

où Z représente un radical méthyle ou éthyle,

c) dans les benzène-sulfonyl-thio-urées substituées par

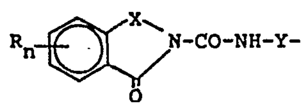

on remplace l'atome de soufre par l'oxygène,

d) on oxyde des benzène-sulfinyl-urées ou des benzène-sulfényl-urées correspondantes,

e) dans des benzène-sulfonyl-urées de formule

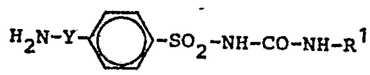

on introduit, éventuellement par étapes, le groupe

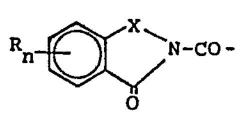

f) on fait réagir des benzènes sulfonyl-halogénures substitués de façon correspondante, avec des urées substituées par $R^1$ ou avec leurs sels alcalins, ou on fait réagir des halogénures de l'acide benzène sulfinique substitués de façon correspondante, ou, en présence d'agents de condensation acides, également des acides sulfiniques substitués de façon correspondante ou leurs sels alcalins, avec une N—$R^1$—N'-hydroxy-urée, et l'on traite éventuellement les produits de réaction avec des agents alcalins pour former des sels.